# EUROPEAN PATENT SPECIFICATION

(11) **EP 3 613 454 B1**
(45) Date of publication and mention of the grant of the patent: **18.08.2021**
(21) Application number: 17906670.9
(22) Date of filing: 05.05.2017
(51) Int. Cl.: A61M 16/04

(54) **AIR CUTTING TYPE BRIDGE FRAME FOR CONTROLLING BALANCED VENTILATION OF SINGLE-CAVITY LUNG ISOLATION CATHETER**
LUFTTRENNENDES BRÜCKENGESTELL ZUR STEUERUNG DER BALANCIERTEN BELÜFTUNG EINES LUNGENISOLATIONSKATHETERS MIT EINZELHOHLRAUM
CADRE PONT DE TYPE COUPE-AIR PERMETTANT DE RÉGULER UNE VENTILATION ÉQUILIBRÉE D'UN CATHÉTER D'ISOLEMENT DE POUMON À UNE SEULE CAVITÉ

(30) Priority: 18.04.2017 CN 201710252634
(43) Date of publication of application: 26.02.2020
(73) Proprietor: Xiao, Jinfang, Guangzhou, Guangdong 510000 (CN)
(72) Inventor: XIAO, Ming, Guangzhou Guangdong 510000 (CN); XIAO, Jinfang, Guangzhou Guangdong 510000 (CN)
(74) Representative: De Lorenzo, Danilo
(86) International application number: PCT/CN2017/083268
(87) International publication number: WO 2018/192013

(56) References cited:
- WO-A1-2005/084739
- CN-A- 104 524 678
- CN-U- 201 558 393
- CN-U- 205 073 462
- CN-U- 205 073 462
- CN-Y- 200 991 488
- US-A1- 2011 186 053

## Description

### Cross-Reference to Related Applications

This application claims priority of Chinese Patent Application No. 201710252634.X, filed with the Chinese Patent Office on April 18, 2017, entitled "Air Cutting Type Bridge Frame for Controlling Balanced Ventilation of Single-Cavity Lung Isolation Catheter".

### Technical Field

The present invention relates to the field of medical appliances, and in particular to a gas-dividing-type single-lumen lung isolation catheter allowing bridge structure span and controlled balanced ventilation (air cutting type bridge frame for controlling balanced ventilation of single-cavity lung isolation catheter).

### Background Art

No breakthrough improvement has been made on a double-lumen (cavity) bronchial catheter (tube) in clinical use for 40 years. The catheter is thick because there is a "separator" in the middle. The main function of the "separator" of the double-lumen bronchial catheter is to isolate the left and right lungs from each other, while the "separator" in the middle ensures balanced ventilation of the left and right lungs. Therefore, the catheter is "thick" due to the presence of the "separator". Due to the defect of its large thickness, the catheter may injure the oral cavity, vocal cords, and tracheal walls; position alignment is required and misalignment easily occurs hence it is necessary to perform the position alignment by intubation with aid of a fiberoptic bronchoscope which is a higher-end instrument; when the body position changes, the catheter is prone to rotational displacement; the catheter cannot meet the requirements of minimally invasive visualizable cardiothoracic surgery in children; most importantly, complete lung collapse or lung atrophy on the operated side is easily caused, and occurrence of acute lung injury or re-expansion pulmonary edema after surgery is increased; the chance of postoperative pulmonary infection or the like is increased; the catheter has many joints, so that cumbersome operations are required in preparation for anesthesia, and the risk of detachment of the joints is increased.

The Chinese patent application CN205073462U discloses a lung isolation catheter device for controlling intrapulmonary equilibrium and gas distribution that forms part of the state of the art.

Ten major defects are summarized as follows: 1. the catheter is thick, so that oral tissues, vocal cords, and tracheal walls may be scratched, and it is necessary to fully expose the oral and pharyngeal cavities; 2. misalignment easily occurs and it is necessary to perform intubation and position alignment with assistance of a fiberoptic bronchoscope; 3. when the body position changes, the catheter is prone to rotational displacement, which affects ventilation and causes a life risk; 4. the catheter is produced with complicated processes and high cost; 5. raw materials are highly consumed in the production of the catheter, which is not environmentally friendly; 6. the catheter has high price, resulting in high medical cost; 7. most importantly, complete lung collapse or lung atrophy on the operated side is easily caused, and the occurrence of acute lung injury, re-expansion pulmonary edema, postoperative pulmonary infection or the like after surgery is increased; 8. the double-lumen tracheal catheter is operated complicatedly and needs to be inserted into a patient without ventilation, which leads to the risk of iatrogenic hypoxia in the patient; 9. gas distribution in the left and right lungs is unbalanced when the oropharyngeal intubation using double-lumen double-opening catheter is operated in such a manner that anatomical alignment is not achieved; 10. bridge structure span cannot be formed in the trachea, and gas advection occurs in the lung, which is one of the factors of normal lung injury caused by mechanical ventilation during anesthesia.

### Summary

The invention is defined in the appended claim 1. Preferred embodiments are matter of the dependent claims.

An object of the present invention is to provide a gas-dividing-type single-lumen lung isolation catheter (configured for) allowing bridge structure span and controlled balanced ventilation, with which the traditional double-lumen tracheal catheter is replaced to solve the problem of the excessively thick outer diameter of the catheter and to solve the technical problem that the prior lung isolation catheter may be unusable since it is easily bent and broken and the technical problem that its using effect is easily affected by a relatively dispersed ventilation (gas) flow at the second perforated region.

The present invention provides a gas-dividing-type single-lumen lung isolation catheter allowing bridge structure span and controlled balanced ventilation, comprising: a main tracheal catheter and a bronchial catheter communicating with one end of the main tracheal catheter, wherein a first opening is provided at an end of the bronchial catheter, and a second perforated region with at least two apertures is provided in a lower end side wall of the main tracheal catheter; the plurality of apertures of the second perforated region are distributed on the same or different transverse sections or on the same or different longitudinal sections in a three-dimensional space formed by the main tracheal catheter.

Optionally, when the second perforated region has three apertures, lines sequentially joining the apertures of the second perforated region with three apertures are distributed in a triangular shape in the three-dimensional space formed by the wall of the main tracheal catheter.

Specifically, when the second perforated region has three apertures, lines joining the three apertures of the second perforated region with three apertures are distributed in a triangular shape in the three-dimensional space formed by the tube wall of the main tracheal catheter, and the triangle is any one of an isosceles triangle, an equilateral triangle, a right triangle, an isosceles right triangle, and a right triangle having an interior angle of 30º, and any other triangle having interior angles having a sum of 180º.

Optionally, when the second perforated region has four apertures, lines sequentially joining the apertures of the second perforated region with four apertures are distributed in a parallelogram shape or any other quadrilateral shape having interior angles totaling 360º in the three-dimensional space formed by the tube wall of the main tracheal catheter, or similarly, when the second perforated region has more than four apertures, lines sequentially joining the apertures of the second perforated region are distributed in a polygonal shape such as a pentagon or hexagon.

Optionally, when the second perforated region has four apertures, two lines joining the respective pair of opposite apertures among the four apertures of the second perforated region are distributed in a cruciform shape in the three-dimensional space formed by the tube wall of the main tracheal catheter.

Specifically, when the second perforated region has four apertures, two lines joining the respective pair of opposite apertures among the four apertures of the second perforated region are distributed in a cruciform shape in the three-dimensional space formed by the tube wall of the main tracheal catheter, and the cruciform shape has a width and a height equal to each other.

In practical applications, each of the plurality of apertures of the second perforated region has an inclined hole structure, and a wall of each aperture of the second perforated region is disposed inclinedly with respect to the side wall of the main tracheal catheter.

Here, the wall of each aperture of the second perforated region is disposed inclinedly with respect to the side wall of the main tracheal catheter and is inclined at an angle ranging from 0º to 90º from a horizontal plane. According to the invention, the inclination angle is a special angle including any one of 5º, 10º, 15º, 30º, 45º, and 60º from the horizontal plane.

In practical applications, a sputum suction hole is provided at the lower end of the main tracheal catheter and adjacent to the bronchial catheter, wherein the sputum suction hole is provided to be facing away from the first opening, and a hole diameter of the sputum suction hole is identical with an opening diameter of the first opening.

Here, a dedicated sputum suction tube passes through the sputum suction hole, wherein the dedicated sputum suction tube is provided with two small holes, and the two small holes are provided oppositely to each other.

Specifically, one end of the dedicated sputum suction tube is disposed in a bent manner and is bent at an angle ranging from 90º to 150º.

Further, a diameter of the dedicated sputum suction tube is 1/3 of a diameter of the bronchial catheter.

In practical applications, a first balloon (airbag) is disposed on an outer wall of the main tracheal catheter and located above the second perforated region, wherein the first balloon communicates with a first inflation valve; a second balloon is disposed on an outer wall of the bronchial catheter, and the second balloon communicates with a second inflation valve; a cuff seal is disposed inside the bronchial catheter, and the cuff seal communicates with a third inflation valve via a ventilation pressure controller; the ventilation pressure controller is used for displaying a pressure in the cuff seal and regulating the size of the cuff seal for blockage to control the ventilation flow volume through the bronchial catheter and to regulate a balanced gas distribution in the lungs.

Here, the first opening has an area of S_{end}, and the plurality of the apertures in the second aperture region have a total area of S; and specifically, S ≥ 1.5Send.

Compared with the prior art, the gas-dividing-type single-lumen lung isolation catheter allowing bridge structure span and controlled balanced ventilation as described in the present invention has the following advantages:

The gas-dividing-type single-lumen lung isolation catheter allowing bridge structure span and controlled balanced ventilation according to the present invention comprises: a main tracheal catheter and a bronchial catheter communicating with one end of the main tracheal catheter, wherein a first opening is provided at an end of the bronchial catheter, and a second perforated region with at least two apertures is provided in a lower end side wall of the main tracheal catheter; the plurality of apertures of the second perforated region are distributed on the same or different transverse sections or longitudinal sections in a three-dimensional space formed by the main tracheal catheter. It can be seen from this analysis that in the gas-dividing-type single-lumen lung isolation catheter allowing bridge structure span and controlled balanced ventilation according to the present invention, the plurality of apertures of the second perforated region provided in the lower end side wall of the main tracheal catheter are distributed on the same or different transverse sections or longitudinal sections, therefore the effect of effectively avoiding bending and breakage of the single-lumen catheter for lung isolation can be achieved without thickening the tube wall of the main tracheal catheter.

In summary, the gas-dividing-type single-lumen lung isolation catheter allowing bridge structure span and controlled balanced ventilation as described in the present invention has the following innovative features: (1) the lower end of the tracheal catheter is perforated in an open manner with two or more apertures in its circumference, and the direction and distribution of the apertures are physically designed to facilitate the balanced gas distribution and the mechanical distribution in a part of the material of the catheter; (2) the electronic ventilation pressure control device can display the pressure in blockage cuffs inside the trachea and on the outer wall of the trachea and can regulate the sizes of the blockage cuffs; (3) regulable blockage cuffs may be disposed in a single lumen of the tracheal catheter and in the bronchial catheter to adjust the ventilation flow volume through the bronchial catheter and the controlled balanced gas distribution in the lungs; (4) the size of a blockage balloon disposed on the outer wall of the single-lumen main tracheal catheter and the size of a blockage balloon disposed on the outer wall of the bronchial catheter are adjusted to form a bridge structure span between the bronchus and the trachea and the carina portion, and the main tracheal catheter is perforated such that the aerodynamic advection effect is changed so as to be more conformable to the physiological mixed-flow ventilation condition; (5) with the above design, the Controlled Balanced Ventilation and Bridge Structure Span (CBV & BSS) mechanism is demonstrated to ensure a minimum effective ventilation volume in single-lung ventilation, reduce damage to the main tracheal and the bronchus, simplify the position alignment, and solve the problem of lung isolation in children; the controlled balanced ventilation allows an effective adjustment of the balance between oxygen supply and oxygen consumption, especially for the problem of adjusting oxygen supply during surgery in an elderly patient with chronic obstructive pulmonary disease (COPD) and hypoxia easily occurring in children, thereby ameliorating pulmonary arterial hypertension after a lung collapse.

### Brief Description of Drawings

In order to more clearly illustrate technical solutions of specific embodiments of a gas-dividing-type single-lumen lung isolation catheter allowing bridge structure span and controlled balanced ventilation according to the present invention or of the prior art, drawings required for use in the description of the specific embodiments or the prior art will be described briefly below. It is obvious that the drawings in the following description are illustrative of some special embodiments of the present invention. It will be understood by those of ordinary skill in the art that other drawings can also be obtained from these drawings without any inventive effort, and any other drawing not shown will fall within the scope of protection of the present invention.

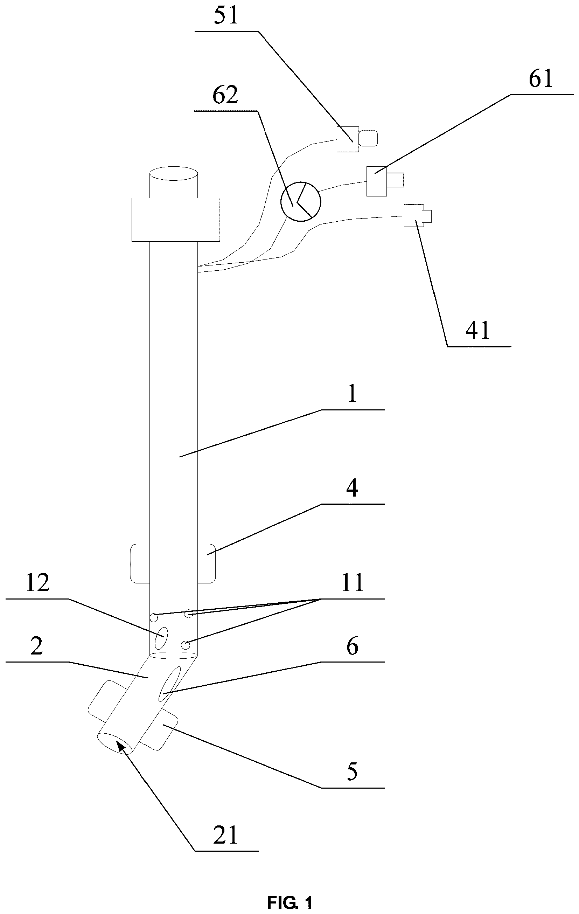

FIG. 1 is a schematic structural view of a gas-dividing-type single-lumen lung isolation catheter allowing bridge structure span and controlled balanced ventilation according to an embodiment of the present invention;

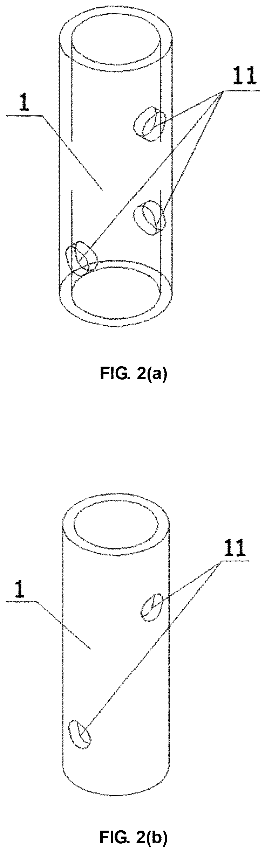

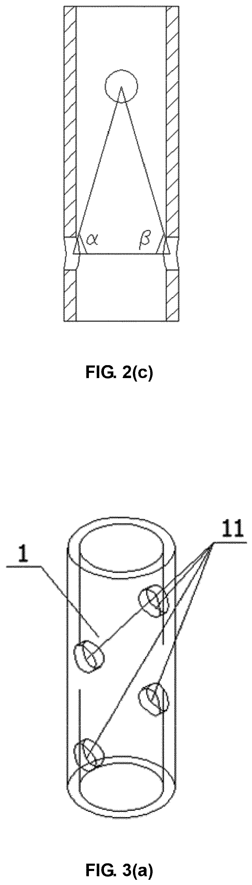

FIG. 2(a), FIG. 2(b), and FIG. 2(c) are schematic three-dimensional and vertically-sectional structural views showing a first distribution of a second perforated region in the gas-dividing-type single-lumen lung isolation catheter allowing bridge structure span and controlled balanced ventilation according to an embodiment of the present invention, respectively;

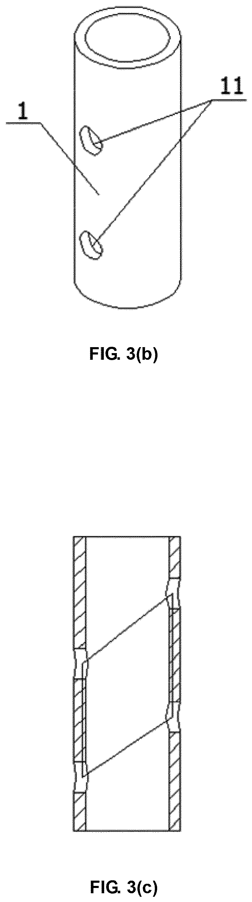

FIG. 3(a), FIG. 3(b), and FIG. 3(c) are schematic three-dimensional and vertically-sectional structural views showing a second distribution of the second perforated region in the gas-dividing-type single-lumen lung isolation catheter allowing bridge structure span and controlled balanced ventilation according to an embodiment of the present invention, respectively;

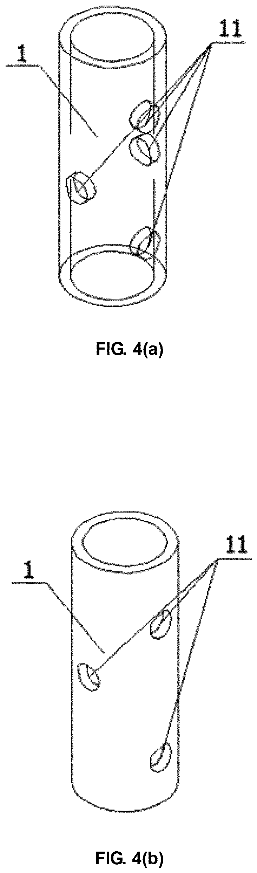

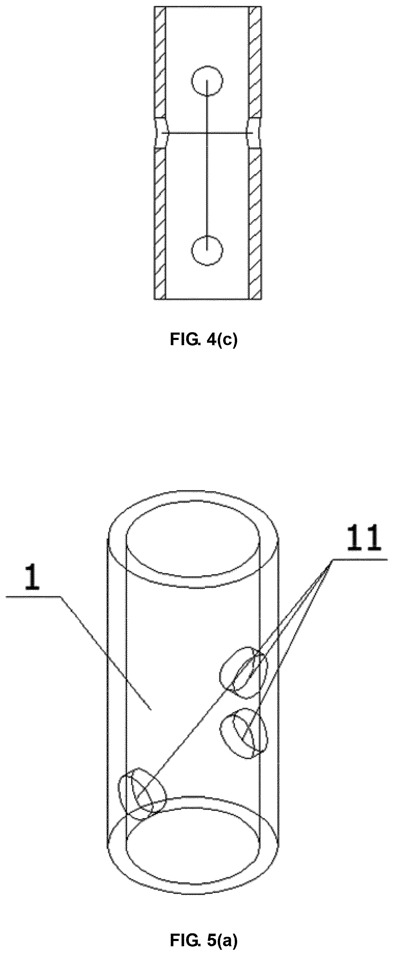

FIG. 4(a), FIG. 4(b), and FIG. 4(c) are schematic three-dimensional and vertically-sectional structural views showing a third distribution of the second perforated region in the gas-dividing-type single-lumen lung isolation catheter allowing bridge structure span and controlled balanced ventilation according to an embodiment of the present invention, respectively;

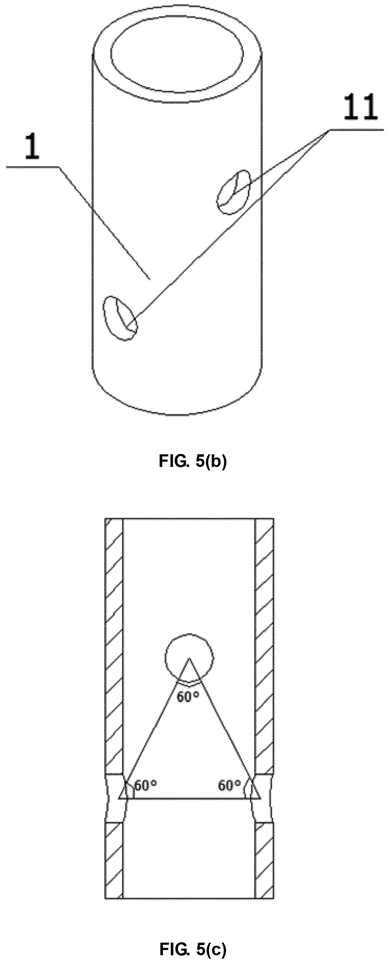

FIG. 5(a), FIG. 5(b), and FIG. 5(c) are schematic three-dimensional and vertically-sectional structural views showing a fourth distribution of the second perforated region in the gas-dividing-type single-lumen lung isolation catheter allowing bridge structure span and controlled balanced ventilation according to an embodiment of the present invention, respectively;

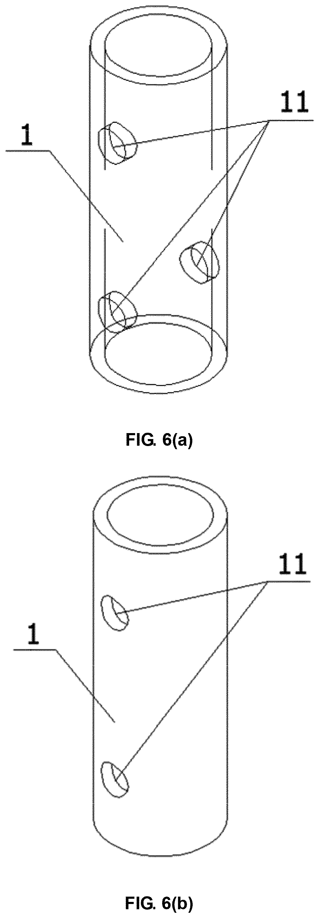

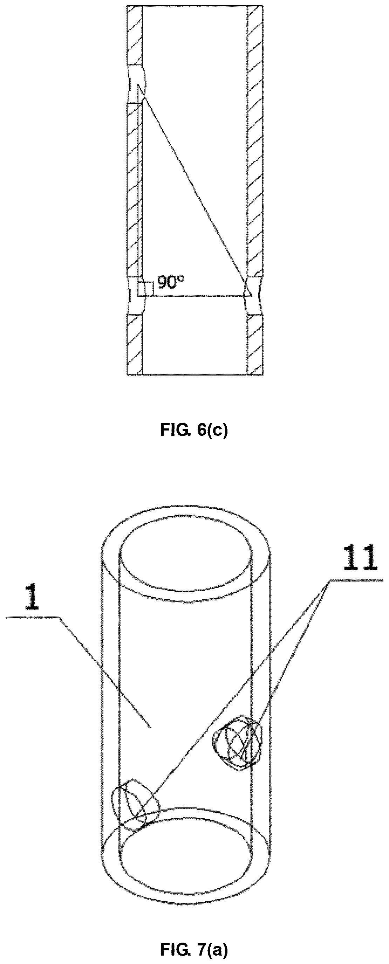

FIG. 6(a), FIG. 6(b), and FIG. 6(c) are schematic three-dimensional and vertically-sectional structural views showing a fifth distribution of the second perforated region in the gas-dividing-type single-lumen lung isolation catheter allowing bridge structure span and controlled balanced ventilation according to an embodiment of the present invention, respectively;

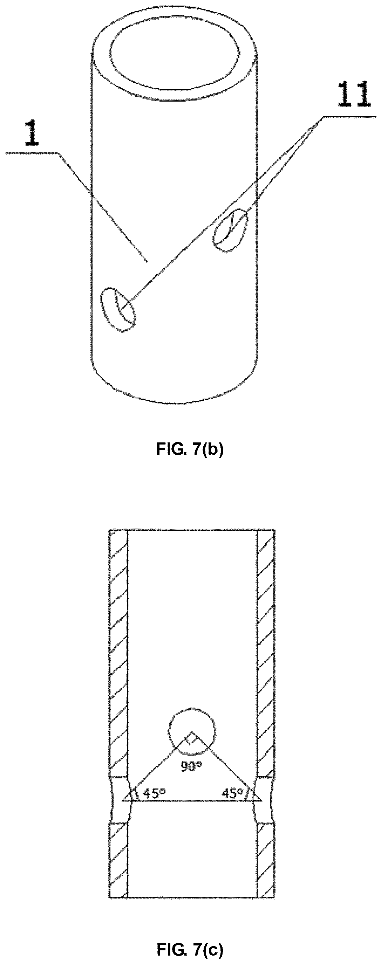

FIG. 7(a), FIG. 7(b), and FIG. 7(c) are schematic three-dimensional and vertically-sectional structural views showing a sixth distribution of the second perforated region in the gas-dividing-type single-lumen lung isolation catheter allowing bridge structure span and controlled balanced ventilation according to an embodiment of the present invention, respectively;

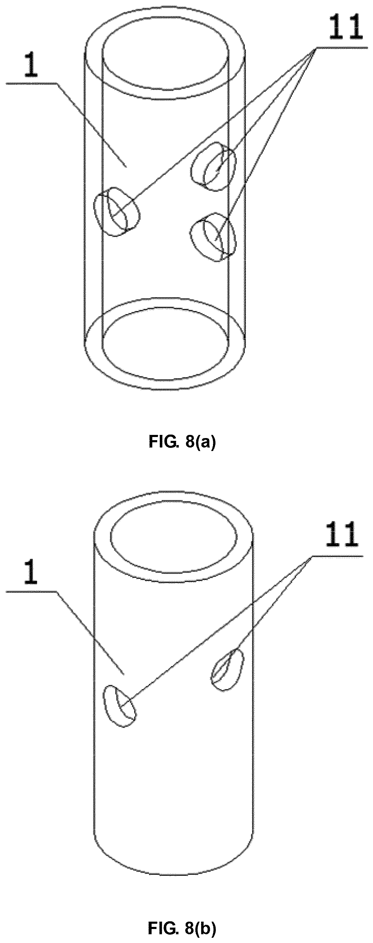

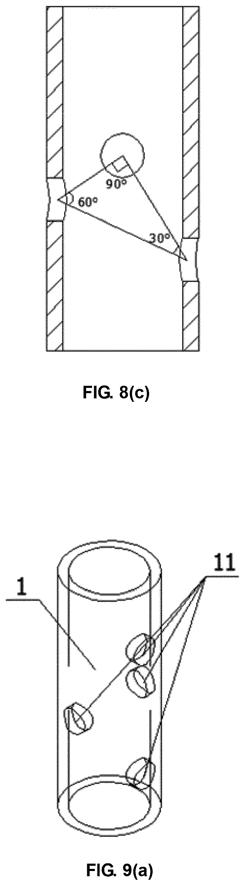

FIG. 8(a), FIG. 8(b), and FIG. 8(c) are schematic three-dimensional and vertically-sectional structural views showing a seventh distribution of the second perforated region in the gas-dividing-type single-lumen lung isolation catheter allowing bridge structure span and controlled balanced ventilation according to an embodiment of the present invention, respectively;

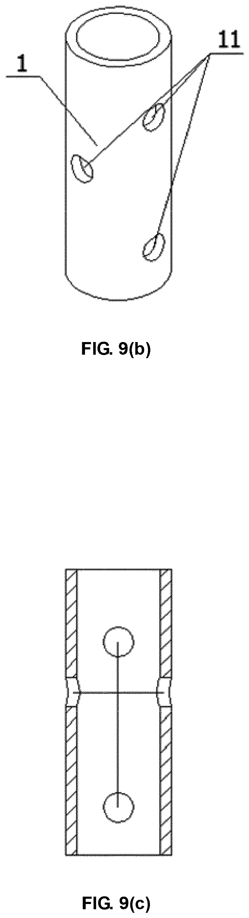

FIG. 9(a), FIG. 9(b), and FIG. 9(c) are schematic three-dimensional and vertically-sectional structural views showing an eighth distribution of the second perforated region in the gas-dividing-type single-lumen lung isolation catheter allowing bridge structure span and controlled balanced ventilation according to an embodiment of the present invention, respectively;

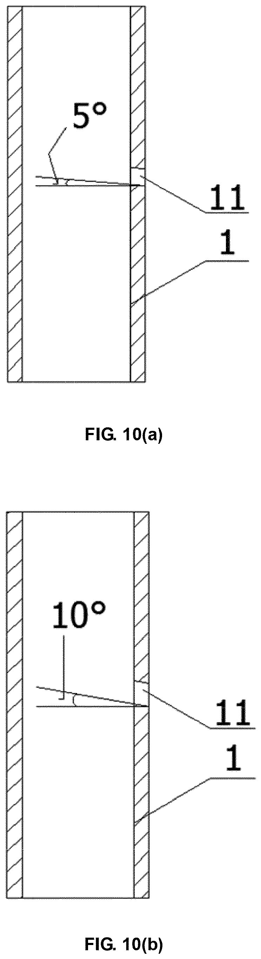

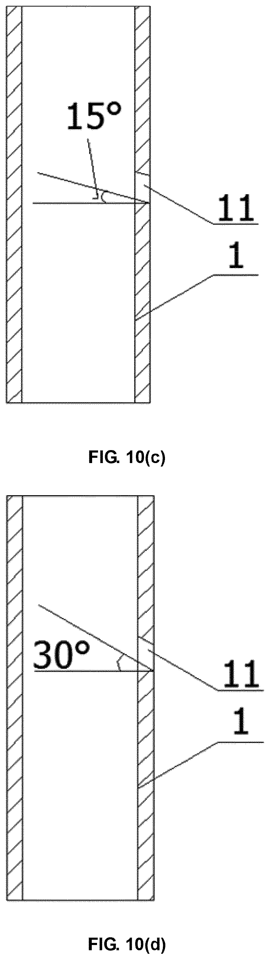

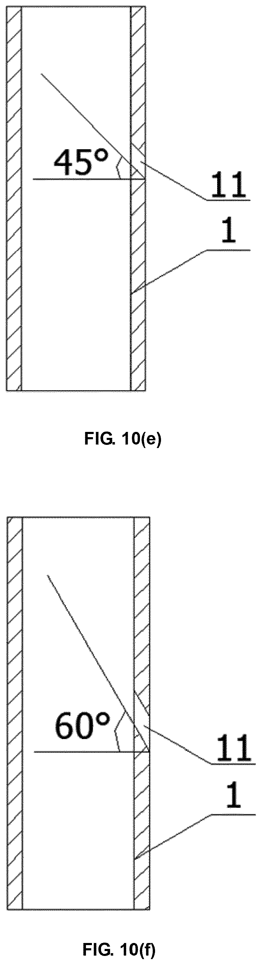

FIG. 10(a) to FIG. 10(f) are schematic vertically-sectional structural views showing an inclination angle of the second perforated region in the gas-dividing-type single-lumen lung isolation catheter allowing bridge structure span and controlled balanced ventilation according to an embodiment of the present invention;

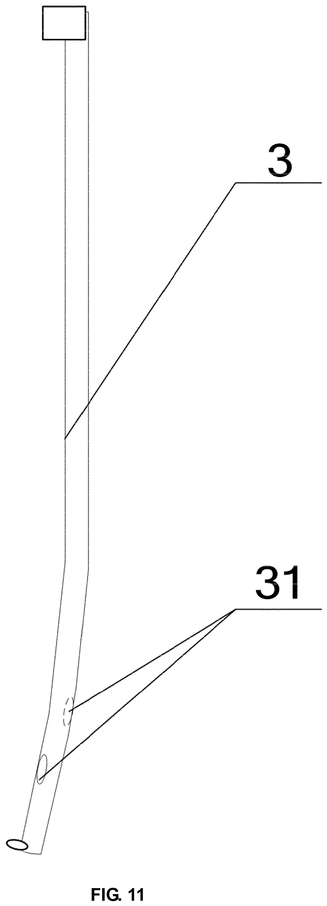

FIG. 11 is a schematic structural view of a dedicated sputum suction tube in the gas-dividing-type single-lumen lung isolation catheter allowing bridge structure span and controlled balanced ventilation according to an embodiment of the present invention;

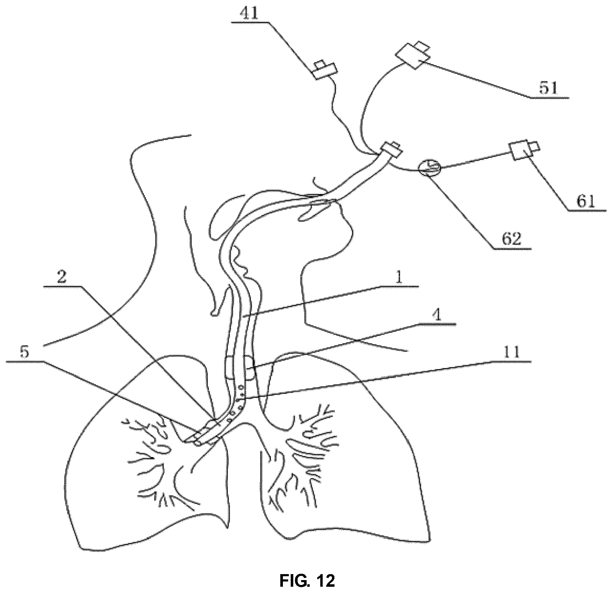

FIG. 12 is a schematic structural view showing use of the gas-dividing-type single-lumen lung isolation catheter allowing bridge structure span and controlled balanced ventilation according to an embodiment of the present invention;

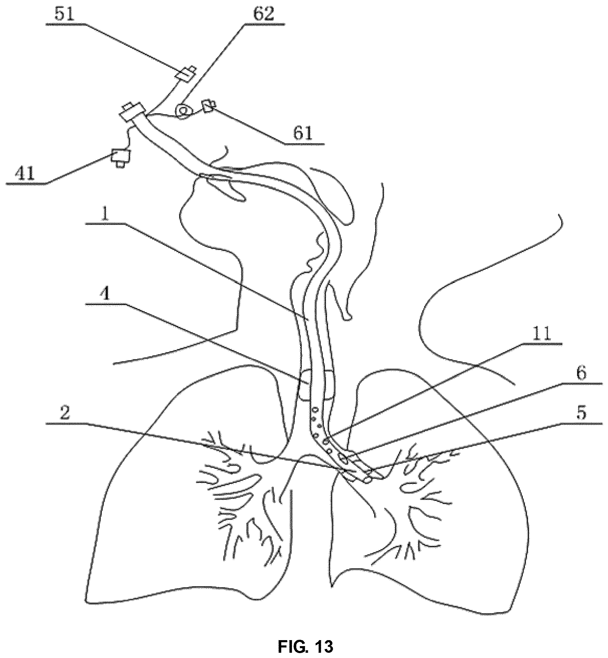

FIG. 13 is another schematic structural view showing use of the gas-dividing-type single-lumen lung isolation catheter allowing bridge structure span and controlled balanced ventilation according to an embodiment of the present invention; and

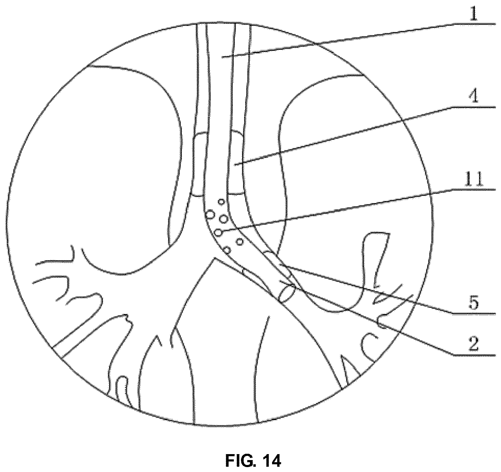

FIG. 14 is a schematic structural view showing balanced pulmonary ventilation and bridge structure span during use of the gas-dividing-type single-lumen lung isolation catheter allowing bridge structure span and controlled balanced ventilation according to an embodiment of the present invention.

In the figures: 1-main tracheal catheter; 2-bronchial catheter; 21-first opening; 11-second perforated region; 12-sputum suction hole; 3-dedicated sputum suction tube; 31-small hole; 4-first balloon; 41-first inflation valve; 5-second balloon; 51-second inflation valve; 6-cuff seal; 61-third inflation valve; 62-ventilation pressure controller.

### Detailed Description of Embodiments

The technical solutions of a gas-dividing-type single-lumen lung isolation catheter allowing bridge structure span and controlled balanced ventilation according to the present invention will be described below clearly and completely with reference to the accompanying drawings. It is apparent that the embodiments to be described are some, but not all of the embodiments of the present invention. All the other embodiments obtained by those of ordinary skill in the art in light of the embodiments of the present invention without inventive efforts will fall within the scope of the present invention as claimed.

In the description of the present invention, it should be noted that orientation or positional relations indicated by the terms such as "center", "up", "down", "left", "right", "vertical", "horizontal", "inside", and "outside" are the orientation or positional relations shown based on the figures, and these terms are intended only to facilitate the description of the present invention and simplify the description, but not intended to indicate or imply that the referred devices or elements must be in a particular orientation or constructed or operated in the particular orientation, and therefore should not be construed as limiting the present invention. In addition, the terms "first", "second", and "third" are used for descriptive purposes only, and should not be understood as an indication or implication of relative importance.

In the description of the gas-dividing-type single-lumen lung isolation catheter allowing bridge structure span and controlled balanced ventilation according to the present invention, it should be noted that the terms "mounted", "coupled", and "connected" should be understood broadly unless otherwise expressly specified or defined. For example, a connection may be fixed connection or detachable connection or integral connection, may be mechanical connection or electrical connection, or may be direct coupling or indirect coupling via intermediate medium or internal communication between two elements. The specific meanings of the above-mentioned terms in the present invention can be understood by those of ordinary skill in the art according to specific situations.

FIG. 1 is a schematic structural view of a gas-dividing-type single-lumen lung isolation catheter allowing bridge structure span and controlled balanced ventilation according to an embodiment of the present invention; FIG. 2(a), FIG. 2(b), and FIG. 2(c) are three-dimensional and vertically-sectional schematic structural views showing a first distribution of a second perforated region in the gas-dividing-type single-lumen lung isolation catheter allowing bridge structure span and controlled balanced ventilation according to an embodiment of the present invention, respectively; FIG. 3(a), FIG. 3(b), and FIG. 3(c) are three-dimensional and vertically-sectional schematic structural views showing a second distribution of the second perforated region in the gas-dividing-type single-lumen lung isolation catheter allowing bridge structure span and controlled balanced ventilation according to an embodiment of the present invention, respectively; and FIG. 4(a), FIG. 4(b), and FIG. 4(c) are three-dimensional and vertically-sectional schematic structural views showing a third distribution of the second perforated region in the gas-dividing-type single-lumen lung isolation catheter allowing bridge structure span and controlled balanced ventilation according to an embodiment of the present invention, respectively.

As shown in FIG. 1 in combination with FIGS. 2(a) to 2(c), FIGS. 3(a) to 3(c), and FIGS. 4(a) to 4(c), an embodiment of the present invention provides a gas-dividing-type single-lumen lung isolation catheter allowing bridge structure span and controlled balanced ventilation, comprising: a main (pipe) tracheal catheter (tube) 1 and a bronchial catheter 2 communicating with one end of the main tracheal catheter 1, wherein a first opening 21 is provided at an end of the bronchial catheter 2, and a second perforated region (aperture region) 11 with at least two apertures is provided in a lower end side wall of the main tracheal catheter 1; the plurality of apertures of the second perforated region 11 are distributed on the same or different transverse sections or on the same or different longitudinal sections in a three-dimensional space formed by the main tracheal catheter 1 (as shown in FIGS. 2(a) to 2(c), FIGS. 3(a) to 3(c), and FIGS. 4(a) to 4(c)).

Compared with the prior art, the gas-dividing-type single-lumen lung isolation catheter allowing bridge structure span and controlled balanced ventilation according to the embodiment of the present invention has the following advantages:
The gas-dividing-type single-lumen lung isolation catheter allowing bridge structure span and controlled balanced ventilation according to the embodiment of the present invention, as shown in FIG. 1 in combination with FIGS. 2(a) to 2(c), FIGS. 3(a) to 3(c), and FIGS. 4(a) to 4(c), comprises: a main tracheal catheter 1 and a bronchial catheter 2 communicating with one end of the main tracheal catheter 1, wherein a first opening 21 is provided at an end of the bronchial catheter 2, and a second perforated region 11 with at least two apertures is provided in a lower end side wall of the main tracheal catheter 1; the plurality of apertures of the second perforated region 11 are distributed on the same or different transverse sections or on the same or different longitudinal sections in a three-dimensional space formed by the main tracheal catheter 1 (as shown in FIGS. 2(a) to 2(c), FIGS. 3(a) to 3(c), and FIGS. 4(a) to 4(c)). It can be seen from this analysis that in the gas-dividing-type single-lumen lung isolation catheter allowing bridge structure span and controlled balanced ventilation according to the embodiment of the present invention, the plurality of apertures of the second perforated region 11 provided in the lower end side wall of the main tracheal catheter 1 are distributed on the same or different transverse sections or on the same or different longitudinal sections, therefore the effect of effectively avoiding bending and breakage of the single-lumen lung isolation catheter can be achieved without thickening the tube wall of the main tracheal catheter 1.

Here, as shown in FIGS. 2(a) to 2(c), when the second perforated region 11 has three apertures, lines sequentially joining the three apertures of the second perforated region 11 are distributed in a triangular shape in the three-dimensional space formed by the tube wall of the main tracheal catheter 1.

Here, in order to further improve the stability of the main tracheal catheter 1 while ensuring the ventilation volume, in actual production and manufacturing, as shown in FIGS. 2(a) to 2(c), when the second perforated region 11 has three apertures, lines joining the three apertures of the second perforated region 11 are distributed in a triangular shape in the three-dimensional space formed by the tube wall of the main tracheal catheter 1, and the triangle may be an isosceles triangle, that is, a triangle with two equal interior angles among the three interior angles thereof. As shown in FIG. 2, an angle a is equal to an angle β.

FIG. 5(a), FIG. 5(b), and FIG. 5(c) are three-dimensional and vertically-sectional schematic structural views showing a fourth distribution of the second perforated region in the gas-dividing-type single-lumen lung isolation catheter allowing bridge structure span and controlled balanced ventilation according to an embodiment of the present invention, respectively.

As shown in FIG. 5, when the second perforated region 11 has three apertures, lines joining the three apertures of the second perforated region 11 are distributed in a triangular shape in the three-dimensional space formed by the tube wall of the main tracheal catheter 1, and the triangle may be an equilateral triangle, that is, a triangle with three interior angles each being 60º.

FIG. 6(a), FIG. 6(b), and FIG. 6(c) are three-dimensional and vertically-sectional schematic structural views showing a fifth distribution of the second perforated region in the gas-dividing-type single-lumen lung isolation catheter allowing bridge structure span and controlled balanced ventilation according to an embodiment of the present invention, respectively.

As shown in FIG. 6, when the second perforated region 11 has three apertures, lines joining the three apertures of the second perforated region 11 are distributed in a triangular shape in the three-dimensional space formed by the tube wall of the main tracheal catheter 1, and the triangle may be a right triangle, that is, a triangle having one interior angle being 90º among three interior angles thereof.

FIG. 7(a), FIG. 7(b), and FIG. 7(c) are three-dimensional and vertically-sectional schematic structural views showing a sixth distribution of the second perforated region in the gas-dividing-type single-lumen lung isolation catheter allowing bridge structure span and controlled balanced ventilation according to an embodiment of the present invention, respectively.

As shown in FIG. 7, when the second perforated region 11 has three apertures, lines joining the three apertures of the second perforated region 11 are distributed in a triangular shape in the three-dimensional space formed by the tube wall of the main tracheal catheter 1, and the triangle may be an isosceles right triangle, that is, a triangle having three interior angles including an interior angle being 90º and two remaining interior angles being 45º.

FIG. 8(a), FIG. 8(b), and FIG. 8(c) are three-dimensional and vertically-sectional schematic structural views showing a seventh distribution of the second perforated region in the gas-dividing-type single-lumen lung isolation catheter allowing bridge structure span and controlled balanced ventilation according to an embodiment of the present invention, respectively.

As shown in FIG. 8, when the second perforated region 11 has three apertures, lines joining the three apertures of the second perforated region 11 are distributed in a triangular shape in the three-dimensional space formed by the tube wall of the main tracheal catheter 1, and the triangle may be a right triangle having an interior angle of 30º, that is, a triangle having three interior angles including an interior angle being 30º, an interior angle being 60º, and an interior angle being 90º.

Evidently, in addition to the special triangles listed above, triangles having random interior angle degrees are also possible, which are not limited herein, but all fall within the scope of protection of the present invention.

As shown in FIGS. 3(a) to 3(c), when the second perforated region 11 has four apertures, lines sequentially joining the four apertures of the second perforated region 11 are distributed in a parallelogram shape in the three-dimensional space formed by the tube wall of the main tracheal catheter 1.

Optionally, as shown in FIGS. 4(a) to 4(c), when the second perforated region 11 has four apertures, two lines joining the respective pair of opposite apertures among the four apertures of the second perforated region 11 are distributed in a cruciform shape in the three-dimensional space formed by the tube wall of the main tracheal catheter 1.

FIG. 9(a), FIG. 9(b), and FIG. 9(c) three-dimensional and vertically-sectional are schematic structural views showing an eighth distribution of the second perforated region in the gas-dividing-type single-lumen lung isolation catheter allowing bridge structure span and controlled balanced ventilation according to an embodiment of the present invention, respectively.

Further, as shown in FIGS. 9(a) to 9(c), when the second perforated region 11 has four apertures, two lines joining the respective pair of opposite apertures among the four apertures of the second perforated region 11 are distributed in a cruciform shape in the three-dimensional space formed by the wall of the main tracheal catheter 1, and the cruciform shape has a width and a height equal to each other. Evidently, the length and the width may also be set to be unequal.

It should be additionally noted here that the number and specific distribution of the apertures of the second perforated region 11 are not limited to the cases enumerated above, and other reasonable distributions are also possible as long as the strength of the tube wall of the main tracheal catheter 1 can be effectively ensured to avoid bending and breakage of the gas-dividing-type single-lumen lung isolation catheter allowing bridge structure span and controlled balanced ventilation. For example, it is also possible that the second perforated region 11 includes four apertures, and lines joining the four apertures of the second perforated region 11 are distributed in a tetrahedral shape in the three-dimensional space formed by the tube wall of the main tracheal catheter 1; or, the second perforated region 11 includes five apertures, and lines joining the five apertures of the second perforated region 11 are distributed in a pentagonal shape in the three-dimensional space formed by the tube wall of the main tracheal catheter 1; or, the second perforated region 11 includes six apertures, and lines joining the six apertures of the second perforated region 11 are distributed in a pentahedral shape in the three-dimensional space formed by the tube wall of the main tracheal catheter 1, and other cases are not exhaustively enumerated here.

In practical applications, in the gas-dividing-type single-lumen lung isolation catheter allowing bridge structure span and controlled balanced ventilation according to the embodiment of the present invention, the plurality of apertures of the second perforated region 11 each have an inclined hole structure, and the hole walls of the second perforated region 11 are disposed inclinedly with respect to the side wall of the main tracheal catheter 1. Since the plurality of apertures of the second perforated region 11 each have an inclined hole structure, that is to say, the hole walls of the second perforated region 11 are disposed inclinedly with respect to the side wall of the main tracheal catheter 1, a ventilating gas flow is more conformable to the physiologically mixed flow state based on the inclination angle and the magnitude of the divided gas volume, so that the capability of gathering the ventilating gas flow is improved, more concentrated ventilating gas flow can be ensured, and the using effect of the single-lumen lung isolation catheter is effectively improved.

Specifically, in the gas-dividing-type single-lumen lung isolation catheter allowing bridge structure span and controlled balanced ventilation according to the embodiment of the present invention, the hole walls of the second perforated region 11 described above are disposed inclinedly with respect to the side wall of the main tracheal catheter 1, and is inclined at an angle of 0º to 90º from a horizontal plane.

FIG. 10(a) to FIG. 10(f) are sectional schematic structural views showing an inclination angle of the second perforated region in the gas-dividing-type single-lumen lung isolation catheter allowing bridge structure span and controlled balanced ventilation according to an embodiment of the present invention.

Further, in order to facilitate processing and manufacturing while ensuring a relatively concentrated ventilating gas flow which flows through the second perforated region 11, as shown in FIGS. 10(a) to 10(f), the inclination angle described above may specifically be 5º, 10º, 15º, 30º, 45º, 60º, or the like. Of co, the specific value of the inclination angle is not limited to the several specific angles described above, and may be any angle between 0º and 90º, which is not exhaustively enumerated here, however according to the present invention the inclination angle is any one of 5°, 10°, 15°, 30°, 45°, 60°.

Moreover, as shown in FIGS. 10(a) to 10(f), in the second perforated region 11, the positions where openings on the inner side wall of the main tracheal catheter 1 are formed are higher than positions where openings on the outer side wall of the main tracheal catheter 1 are formed, so as to facilitate better ventilation.

In practical applications, in order to facilitate a sputum (or secretions) suction operation, in the gas-dividing-type single-lumen lung isolation catheter allowing bridge structure span and controlled balanced ventilation according to the embodiment of the present invention, as shown in FIG. 1, a sputum suction hole 12 may be provided at the lower end of the above-mentioned main tracheal catheter 1 and adjacent to the bronchial catheter 2, the sputum suction hole 12 is provided to face away from the first opening 21, and the hole diameter of the sputum suction hole 12 is preferably identical with the opening diameter of the first opening 21.

It should be additionally noted here that the sputum suction hole 12 should not have an excessively small area, otherwise a dedicated sputum suction tube cannot pass therethrough, resulting in a poor sputum suction effect; and also, the sputum suction hole 12 should not have an excessively large area, otherwise poor mechanical properties are caused, resulting in fracture of the gas-dividing-type single-lumen lung isolation catheter allowing bridge structure span and controlled balanced ventilation in the patient's lung.

FIG. 11 is a schematic structural view of a dedicated sputum suction tube in the gas-dividing-type single-lumen lung isolation catheter allowing bridge structure span and controlled balanced ventilation according to an embodiment of the present invention.

Here, as shown in FIG. 1 in combination with FIG. 11, a dedicated sputum suction tube 3 may pass through the sputum suction hole 12 described above, wherein the dedicated sputum suction tube 3 is provided with two small holes 31, and the two small holes 31 may be provided oppositely to each other to balance the gas pressure.

Specifically, in order to facilitate the insertion of the dedicated sputum suction tube 3 into the gas-dividing-type single-lumen lung isolation catheter allowing bridge structure span and controlled balanced ventilation, as shown in FIG. 11, one end of the dedicated sputum suction tube 3 described above may be disposed in a bent manner and bent at an angle preferably ranging from 90º to 150º.

Further, in actual production and manufacturing, the bending angle described above may be 90º, 120º, or 150º. Evidently, the bending angle is not limited to the above several special angles, and may be any of other reasonable angles, which are not exhaustively enumerated here.

Still further, in order to ensure the sputum suction effect while facilitate the insertion and removal of the dedicated sputum suction tube 3, the diameter of the sputum suction tube 3 may preferably be 1/3 of the diameter of the bronchial catheter 2.

FIG. 12 is a schematic structural view showing use of the gas-dividing-type single-lumen lung isolation catheter allowing bridge structure span and controlled balanced ventilation according to an embodiment of the present invention; FIG. 13 is another schematic structural view showing use of the gas-dividing-type single-lumen lung isolation catheter allowing bridge structure span and controlled balanced ventilation according to an embodiment of the present invention; and FIG. 14 is a schematic structural view showing balanced pulmonary ventilation and bridge structure span during use of the gas-dividing-type single-lumen lung isolation catheter allowing bridge structure span and controlled balanced ventilation according to an embodiment of the present invention.

In practical applications, in the gas-dividing-type single-lumen lung isolation catheter allowing bridge structure span and controlled balanced ventilation according to the embodiment of the present invention, as shown in FIG. 1 in combination with FIG. 12 to FIG. 14, a first balloon 4 is disposed on an outer wall of the main tracheal catheter 1 and the first balloon is located above the second perforated region 11, and the first balloon 4 communicates with a first inflation valve (gas intake valve) 41; a second balloon 5 is disposed on an outer wall of the bronchial catheter 2, and the second balloon 5 communicates with a second inflation valve 51; a cuff seal 6 is disposed inside the bronchial catheter 2, and the cuff seal 6 communicates with a third inflation valve 61 via a ventilation pressure controller 62; wherein ventilation pressure controller 62 is used for displaying a pressure in the cuff seal 6 and regulating the size of the cuff seal 6 for blockage so as to control the ventilation flow volume through the bronchial catheter 2 and to regulate the balanced gas distribution in the lungs.

Such arrangement can achieve the following several advantageous effects:
1) all the functions of a double-lumen tracheal catheter can be achieved, thereby overcoming the disadvantage that the double-lumen bronchial catheter does not facilitate endotracheal intubation and easily causes damage to the trachea;
2) due to a displacement of the tracheal catheter during surgery, the second perforated region is provided in the tracheal catheter so as to avoid the problem of poor ventilation caused by blockage of the apertures of the second perforated region when the tracheal catheter is rotated during the surgery;
3) the size of each aperture if the second perforated region may be set scientifically and practically, so that the catheter body is less likely to be bent, thereby improving the stability of the intubation and solving the problem that a single-hole catheter body is easily bent due to its large hole diameter;
4) after the first balloon and the second balloon are inflated, a structure having both ends protruded is formed, so that the opening portion is in the state of being suspended away from the trachea, bronchus and carina portions, which is called a bridge structure span state; a rotational displacement of the catheter due to the change of the patient's body position is effectively avoided during the surgery to prevent the openings of the catheter from contacting the tracheal wall and the bronchial wall; in addition, a plurality of apertures are provided in the second perforated region, so that the gas flow can be divided to reduce an impact from the gas flow and avoid the advection of the gas, and the displacement of the catheter is more effectively avoided by cooperation with two balloons;
5) the sputum suction tube is more conveniently inserted to facilitate sputum suction; or the second perforated region may be selected and used for sputum suction operated with a dual-hole catheter;
6) the cuff seal in the bronchial catheter is a spherical balloon embedded in the wall of the catheter, and the cuff seal is connected with a third inflation valve and is connected with a ventilation pressure controller capable of displaying the pressure inside the cuff seal and controlling the size of the cuff seal so as to achieve the purpose of controlling the ventilating flow volume through the bronchus and regulating the balanced gas distribution in the lungs, which can meet a long-lasting surgery, guarantee an controllable area of collapse of the lung on the operated side, guarantee the optimal satisfactory operable space, and prevent the complications of re-expansion pulmonary edema, acute lung injury, and lung infection, which is referred to as "controlled balanced ventilation".

It should be additionally noted that, in the gas-dividing-type single-lumen lung isolation catheter allowing bridge structure span and controlled balanced ventilation according to the embodiments of the present invention, as shown in FIG. 1 to FIG. 14, each of the main tracheal catheter 1, the bronchial catheter 2, and the sputum suction tube 3 is a circular tube; and each of the first opening 21, the apertures of the second perforated region 11, and the small holes 31 may be a circular opening or aperture. Evidently, each of the above-mentioned small holes 31 may also be in any other shape, which is not limited herein.

Here, the above-mentioned first opening 21 has an area of S_{end}, and the plurality of apertures in the second perforated region 11 have a total area of S; and specifically, S ≥ 1.5*S_{end}.

For example, two apertures are provided in the second perforated region 11, wherein each aperture s in a circular or elliptical form, and have an aperture area designed based on the aerodynamic physical principle to satisfy the controlled balanced gas distribution in the lungs, that is, an area S1+S2 ≥ 1.5S_{end}. The tidal volumes in the left and right lungs generated by the gas distribution are balanced and meet physiological standards. Taking a person with a standard weight of 60 kg as an example, the tidal volume in the left lung is TV_{left} and the tidal volume in the right lung is TV_{right}, where TV_{left} = TV_{right} = 200 to 370 ml. Balanced ventilation of the left and right lungs is achieved based on the rule of regulating S1+S2 ≥ 1.5S_{end} by apertures for balanced gas distribution in the lungs. Other cases can be deduced similarly based on the weight.

Three apertures are provided in the second perforated region 11 and each have an aperture area designed based on the aerodynamic physical principle to satisfy the controlled balanced gas distribution in the lungs, that is, an area S1 +S2+S3 ≥ 1.5S_{end}. The tidal volumes in the left and right lungs generated by the gas distribution are balanced and meet physiological standards. Taking a person with a standard weight of 60 kg as an example, the tidal volume in the left lung is TV_{left} and the tidal volume in the right lung is TV_{right}, where TV_{left} = TV_{right} = 200 to 370 ml. Balanced ventilation of the left and right lungs is achieved based on the rule of regulating S1+S2+S3 ≥ 1.5Send by apertures for balanced gas distribution in the lungs. Other cases can be deduced similarly based on the weight.

Four apertures are provided in the second perforated region 11 and each have an aperture area designed based on the aerodynamic physical principle to satisfy the controlled balanced gas distribution in the lungs, that is, an area S1+S2+S3+S4 ≥ 1.5S_{end}. The tidal volumes in the left and right lungs generated by the gas distribution are balanced and meet physiological standards. Taking a person with a standard weight of 60 kg as an example, the tidal volume in the left lung is TV_{left} and the tidal volume in the right lung is TV_{right}, where TV_{left} = TV_{right} = 200 to 370 ml. Balanced ventilation of the left and right lungs is achieved based on the rule of regulating S1+S2+S3+S4 ≥ 1.5S_{end} by apertures for balanced gas distribution in the lungs. Other cases can be deduced similarly based on the weight.

In addition, cases, in which more than four apertures are provided in the second perforated region 11 and or the second perforated region is perforated in other manners, the rest can be deduced similarly.

The gas-dividing-type single-lumen lung isolation catheter allowing bridge structure span and controlled balanced ventilation according to the embodiments of the present invention is used for solving and improving the efficiency of balanced ventilation of the left and right lungs in a thoracic surgery using a novel lung isolation catheter. A geometrical distribution of perforations in the tube wall of the gas-dividing-type single-lumen lung isolation catheter allowing bridge structure span and controlled balanced ventilation is used for solving the problem that the catheter may be unusable since it is easily bent and broken, and the catheter supporting strength of the second perforated region is enhanced by the geometrical distribution. The above two techniques are used in conjunction with each other, so that the ventilation flow formed by the gas-dividing-type single-lumen lung isolation catheter allowing bridge structure span and controlled balanced ventilation in the present invention is more conformable to the respiratory physiology for a patient, and the strength of the perforated portions and the compliance of the catheter are enhanced.

The purpose of the present invention is to replace the double-lumen bronchial catheter which is currently in clinical use. The theoretical basis of the single-lumen lung isolation catheter allowing bridge structure span and controlled balanced ventilation is of the concept of Controlled (lung isolation and) Balanced Ventilation and Bridge Structure Span (CBV & BSS), the core of which is the study of a practical catheter tool that can meet the effectiveness of lung isolation and balanced ventilation in clinical anesthesia, and can control the atrophy or collapse of the target lung while the catheter can be held in a suspended state in the trachea and bronchus and carina portions and regions to form a bridge structure span, whereby the position alignment can be simplified, and the diameter of the traditional catheter can be effectively reduced, and moreover a cross-sectional area for ventilation can be enlarged, the functions of the double-lumen bronchial catheter can be replaced and expanded, the requirements for lung isolation in children can be met, and ventilation during surgeries under anesthesia for special patients such as patients with COPD (Chronic Obstructive Pulmonary Disease) can also be improved.

The above description is merely illustrative of preferred embodiments of the present invention and is not intended to limit the present invention. Any modifications, equivalent alternatives, improvements and so on are to be included within the scope of protection of the present invention, which is defined by the claims which follow.

## Claims

1. A gas-dividing-type single-lumen lung isolation catheter configured for allowing bridge structure span and controlled balanced ventilation, comprising: a main tracheal catheter (1) and a bronchial catheter (2) communicating with one end of the main tracheal catheter (1), wherein a tail end of the bronchial catheter (2) is provided with a first opening (21), and a second perforated region (11) with at least two apertures is provided in a lower end side wall of the main tracheal catheter (1), and the at least two apertures of the second perforated region (11) are distributed on same or different transverse sections or on same or different longitudinal sections in a three-dimensional space defined by the main tracheal catheter (1); **characterized in that** each of the at least two apertures of the second perforated region (11) is in an inclined hole structure, and **in that** a wall of the aperture of the second perforated region (11) is disposed inclinedly with respect to the side wall of the main tracheal catheter (1) and has an inclination angle from the horizontal plane of any one of 5º, 10º, 15º, 30º, 45º, and 60º.

2. The gas-dividing-type single-lumen lung isolation catheter configured for allowing bridge structure span and controlled balanced ventilation according to claim 1, wherein when the second perforated region (11) has three apertures, lines sequentially joining the apertures of the second perforated region (11) with the three apertures form a triangle in the three-dimensional space defined by a tube wall of the main tracheal catheter (1).

3. The gas-dividing-type single-lumen lung isolation catheter configured for allowing bridge structure span and controlled balanced ventilation according to claim 2, wherein the triangle is any one of isosceles triangle, equilateral triangle, right triangle, isosceles right triangle, and right triangle having an interior angle of 30º.

4. The gas-dividing-type single-lumen lung isolation catheter configured for allowing bridge structure span and controlled balanced ventilation according to any one of claims 1 to 3, wherein when the second perforated region (11) has three apertures, lines joining the three apertures of the second perforated region (11) with the three apertures form a triangle in the three-dimensional space defined by the tube wall of the main tracheal catheter (1), and the triangle has interior angles totaling 180°.

5. The gas-dividing-type single-lumen lung isolation catheter configured for allowing bridge structure span and controlled balanced ventilation according to claim 1, wherein when the second perforated region (11) has four apertures, lines sequentially joining the apertures of the second perforated region (11) with the four apertures form a parallelogram or any other quadrilateral shape in the three-dimensional space defined by a tube wall of the main tracheal catheter (1).

6. The gas-dividing-type single-lumen lung isolation catheter configured for allowing bridge structure span and controlled balanced ventilation according to claim 1, wherein when the second perforated region (11) has four apertures, two lines joining the respective pair of opposite apertures among the four apertures of the second perforated region (11) form a cruciform shape or any diagonally crossed shape in the three-dimensional space defined by a tube wall of the main tracheal catheter (1).

7. The gas-dividing-type single-lumen lung isolation catheter configured for allowing bridge structure span and controlled balanced ventilation according to claim 6, wherein the cruciform shape has a width and a height equal to each other or the cruciform shape is asymmetrical in width and height.

8. The gas-dividing-type single-lumen lung isolation catheter configured for allowing bridge structure span and controlled balanced ventilation according to claim 1, wherein the wall of each aperture of the second perforated region (11) is disposed inclinedly with respect to the side wall of the main tracheal catheter and has an inclination angle of any one of 5°, 10°, 15°, 30°, 45°, and 60° from a horizontal plane (1).

9. The gas-dividing-type single-lumen lung isolation catheter configured for allowing bridge structure span and controlled balanced ventilation according to any one of claims 1 to 8, wherein a sputum suction hole (12) is provided at a lower end of the main tracheal catheter (1) and adjacent to the bronchial catheter (2), the sputum suction hole (12) is provided to face away from the first opening (21), and a hole diameter of the sputum suction hole (12) is identical with an opening diameter of the first opening (21).

10. The gas-dividing-type single-lumen lung isolation catheter configured for allowing bridge structure span and controlled balanced ventilation according to claim 9, wherein the sputum suction hole (12) is configured to allow a dedicated sputum suction tube (3) to pass therethrough, wherein the dedicated sputum suction tube (3) is provided with two small holes (31), and the two small holes (31) are provided oppositely to each other.

11. The gas-dividing-type single-lumen lung isolation catheter configured for allowing bridge structure span and controlled balanced ventilation according to claim 10, wherein the dedicated sputum suction tube (3) has one end provided to be bent at a bent angle ranging from 90º to 150º; and
preferably, a diameter of the dedicated sputum suction tube (3) is 1/3 of a diameter of the bronchial catheter (2).

12. The gas-dividing-type single-lumen lung isolation catheter configured for allowing bridge structure span and controlled balanced ventilation according to any one of claims 1 to 11, wherein a first balloon (4) is disposed on an outer wall of the main tracheal catheter (1) and located above the second perforated region (11), wherein the first balloon (4) communicates with a first inflation valve (41);
a second balloon (5) is disposed on an outer wall of the bronchial catheter (2), wherein the second balloon (5) communicates with a second inflation valve (51);
a cuff seal (6) is disposed inside the bronchial catheter (2) of the gas-dividing-type single-lumen lung isolation catheter configured for allowing bridge structure span and controlled balanced ventilation, and the cuff seal (6) communicates with a third inflation valve (61) via a ventilation pressure controller (62); and
the ventilation pressure controller (62) is configured for displaying a pressure in the cuff seal (6) and regulating a size of the cuff seal (6) for blockage to control a ventilating flow volume of the bronchial catheter (2) and to regulate a balanced gas distribution in lungs.

13. The gas-dividing-type single-lumen lung isolation catheter configured for allowing bridge structure span and controlled balanced ventilation according to claim 12, wherein the first opening (21) has an area of S_{end}, and the at least two second apertures have a total area of S,
wherein S ≥ 1.5Send.

## Patentansprüche

1. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, umfassend: einen Haupttrachealkatheter (1) und einen Bronchialkatheter (2), welcher mit einem Ende des Haupttrachealkatheters (1) in Verbindung steht, wobei ein auslaufendes Ende des Bronchialkatheters (2) mit einer ersten Öffnung (21) bereitgestellt ist und ein zweiter perforierter Bereich (11) mit wenigstens zwei Aperturen in einer Seitenwand eines unteren Endes des Haupttrachealkatheters (1) bereitgestellt ist und wobei die wenigstens zwei Aperturen des zweiten perforierten Bereichs (11) an gleichen oder verschiedenen transversalen Abschnitten oder an gleichen oder verschiedenen longitudinalen Abschnitten in einem durch den Haupttrachealkatheter (1) definierten, dreidimensionalen Raum verteilt sind; **dadurch gekennzeichnet, dass** jede der wenigstens zwei Aperturen des zweiten perforierten Bereichs (11) in einer geneigten Lochstruktur vorliegt und dass eine Wand der Apertur des zweiten perforierten Bereichs (11) in Bezug auf die Seitenwand des Haupttrachealkatheters (1) geneigt angeordnet ist und einen Neigungswinkel von der horizontalen Ebene von jeglichem aus 5°, 10°, 15°, 30°, 45° und 60° aufweist.

2. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach Anspruch 1, wobei, wenn der zweite perforierte Bereich (11) drei Aperturen aufweist, Linien, welche die Aperturen des zweiten perforierten Bereichs (11) sequenziell mit den drei Aperturen verbinden, in dem durch eine Rohrwand des Haupttrachealkatheters (1) definierten, dreidimensionalen Raum ein Dreieck bilden.

3. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach Anspruch 2, wobei das Dreieck jegliches ist aus einem gleichschenkligen Dreieck, einem gleichseitigen Dreieck, einem rechtwinkligen Dreieck, einem gleichschenkligen, rechtwinkligen Dreieck und einem rechtwinkligen Dreieck mit einem Innenwinkel von 30°.

4. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach einem der Ansprüche 1 bis 3, wobei, wenn der zweite perforierte Bereich (11) drei Aperturen aufweist, Linien, welche die drei Aperturen des zweiten perforierten Bereichs (11) mit den drei Aperturen verbinden, in dem durch die Rohrwand des Haupttrachealkatheters (1) definierten, dreidimensionalen Raum ein Dreieck bilden und das Dreieck Innenwinkel mit insgesamt 180° aufweist.

5. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach Anspruch 1, wobei, wenn der zweite perforierte Bereich (11) vier Aperturen aufweist, Linien, welche die Aperturen des zweiten perforierten Bereichs (11) sequenziell mit den vier Aperturen verbinden, in dem durch eine Rohrwand des Haupttrachealkatheters (1) definierten, dreidimensionalen Raum ein Parallelogramm oder jegliche andere vierseitige Form bilden.

6. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach Anspruch 1, wobei, wenn der zweite perforierte Bereich (11) vier Aperturen aufweist, zwei Linien, welche unter den vier Aperturen des zweiten perforierten Bereichs (11) das jeweilige Paar gegenüberliegender Aperturen verbinden, in dem durch eine Rohrwand des Haupttrachealkatheters (1) definierten, dreidimensionalen Raum eine Kreuzform oder jegliche diagonal gekreuzte Form bilden.

7. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach Anspruch 6, wobei die Kreuzform eine Breite und eine Höhe aufweist, welche einander gleich sind, oder die Kreuzform in Bezug auf Breite und Höhe asymmetrisch ist.

8. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach Anspruch 1, wobei die Wand jeder Apertur des zweiten perforierten Bereichs (11) in Bezug auf die Seitenwand des Haupttrachealkatheters geneigt angeordnet ist und einen Neigungswinkel von jeglichem aus 5°, 10°, 15°, 30°, 45° und 60° von einer horizontalen Ebene (1) aufweist.

9. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach einem der Ansprüche 1 bis 8, wobei ein Sputumsaugloch (12) an einem unteren Ende des Haupttrachealkatheters (1) und benachbart zu dem Bronchialkatheter (2) bereitgestellt ist, wobei das Sputumsaugloch (12) derart bereitgestellt ist, dass es weg von der ersten Öffnung (21) weist, und ein Lochdurchmesser des Sputumsauglochs (12) identisch zu einem Öffnungsdurchmesser der ersten Öffnung (21) ist.

10. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach Anspruch 9, wobei das Sputumsaugloch (12) dazu eingerichtet ist, zu ermöglichen, dass ein zweckbestimmtes Sputumsaugrohr (3) dadurch hindurchtritt, wobei das zweckbestimmte Sputumsaugrohr (3) mit zwei kleinen Löchern (31) bereitgestellt ist und die zwei kleinen Löcher (31) entgegengesetzt zueinander bereitgestellt sind.

11. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach Anspruch 10, wobei das zweckbestimmte Sputumsaugrohr (3) ein Ende aufweist, welches derart bereitgestellt ist, dass es mit einem Biegewinkel gebogen ist, welcher von 90° bis 150° reicht; und
vorzugsweise ein Durchmesser des zweckbestimmten Sputumsaugrohrs (3) 1/3 eines Durchmessers des Bronchialkatheters (2) ist.

12. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach einem der Ansprüche 1 bis 11, wobei ein erster Ballon (4) an einer äußeren Wand des Haupttrachealkatheters (1) angeordnet ist und oberhalb des zweiten perforierten Bereichs (11) platziert ist, wobei der erste Ballon (4) mit einem ersten Aufblasventil (41) in Verbindung steht;
ein zweiter Ballon (5) an einer äußeren Wand des Bronchialkatheters (2) angeordnet ist, wobei der zweite Ballon (5) mit einem zweiten Aufblasventil (51) in Verbindung steht;
eine Manschettendichtung (6) innerhalb des Bronchialkatheters (2) des einlumigen Lungenisolationskatheters vom gasteilenden Typ, welcher dazu eingerichtet ist, eine Brückenstrukturüberspannung und eine gesteuerte/geregelte balancierte Belüftung zu ermöglichen, angeordnet ist und die Manschettendichtung (6) über eine Belüftungsdruck-Steuer-/Regeleinheit (62) mit einem dritten Aufblasventil (61) in Verbindung steht; und
die Belüftungsdruck-Steuer-/Regeleinheit (62) dazu eingerichtet ist, einen Druck in der Manschettendichtung (6) anzuzeigen und eine Größe der Manschettendichtung (6) für eine Blockade zu regulieren, um ein Belüftungsströmungsvolumen des Bronchialkatheters (2) zu steuern und eine balancierte Gasverteilung in Lungen zu regulieren.

13. Einlumiger Lungenisolationskatheter vom gasteilenden Typ, welcher dazu eingerichtet ist, eine Brückenstrukturüberspannung und eine gesteuerte/geregelte balancierte Belüftung zu ermöglichen, nach Anspruch 12, wobei die erste Öffnung (21) eine Fläche von S_{end} aufweist und die wenigstens zwei zweiten Aperturen eine Gesamtfläche von S aufweisen,
wobei S ≥ 1,5Send.

## Revendications

1. Cathéter d'isolement de poumon à une seule lumière de type diviseur de gaz, configuré pour permettre une portée de structure de pont et une ventilation équilibrée commandée, comprenant : un cathéter principal de trachée (1) et un cathéter bronchial (2) en communication avec une extrémité du cathéter principal de trachée (1), une extrémité arrière du cathéter bronchial (2) étant pourvue d'une première ouverture (21), et une deuxième zone perforée (11) avec au moins deux ouvertures étant disposée dans une paroi latérale du côté de l'extrémité inférieure du cathéter principal de trachée (1), et lesdites au moins deux ouvertures de la deuxième zone perforée (11) étant reparties sur les mêmes ou sur différentes parties transversales ou sur les mêmes ou sur différentes parties longitudinales dans un espace tridimensionnel défini par le cathéter principal de trachée (1), **caractérisé en ce que** chacune desdites au moins deux ouvertures de la deuxième zone perforée (11) présente une structure de trou inclinée et **en ce qu'**une paroi de l'ouverture de la deuxième zone perforée (11) est disposée de façon inclinée par rapport à la paroi latérale du cathéter principal de trachée (1) et présente un angle d'inclinaison par rapport au plan horizontal de l'un quelconque de 5°, 10°, 15°, 30°, 45° et 60°.

2. Cathéter d'isolement de poumon à une seule lumière de type diviseur de gaz, configuré pour permettre une portée de structure de pont et une ventilation équilibrée commandée, selon la revendication 1, **caractérisé en ce que** la deuxième zone perforée (11) comprend trois ouvertures, des lignes reliant successivement les ouvertures de la deuxième zone perforée (11) avec les trois ouvertures forment un triangle dans l'espace tridimensionnel défini par une paroi de tube du cathéter principal de trachée (1).

3. Cathéter d'isolement de poumon à une seule lumière de type diviseur de gaz, configuré pour permettre une portée de structure de pont et une ventilation équilibrée commandée, selon la revendication 2, **caractérisé en ce que** le triangle est l'un quelconque parmi des triangles isocèles, un triangle équilatéral, un triangle rectangle, un triangle isocèle rectangle, et un triangle rectangle ayant un angle intérieur de 30°.

4. Cathéter d'isolement de poumon à une seule lumière de type diviseur de gaz, configuré pour permettre une portée de structure de pont et une ventilation équilibrée commandée, selon l'une des revendications 1 à 3, **caractérisé en ce que**, lorsque la deuxième zone perforée (11) comprend trois ouvertures, des lignes reliant les trois ouvertures de la deuxième zone perforée (11) avec les trois ouvertures forment un triangle dans l'espace tridimensionnel défini par la paroi de tube du cathéter principal de trachée (1) et le triangle présente des angles intérieurs d'un total de 180°.

5. Cathéter d'isolement de poumon à une seule lumière de type diviseur de gaz, configuré pour permettre une portée de structure de pont et une ventilation équilibrée commandée, selon la revendication 1, **caractérisé en ce que**, lorsque la deuxième zone perforée (11) comprend quatre ouvertures, des lignes reliant les ouvertures de la deuxième zone perforée (11) avec les quatre ouvertures forment un parallélogramme ou toute autre forme quadrilatérale dans l'espace tridimensionnel défini par une paroi de tube du cathéter principal de trachée (1).

6. Cathéter d'isolement de poumon à une seule lumière de type diviseur de gaz, configuré pour permettre une portée de structure de pont et une ventilation équilibrée commandée, selon la revendication 1, **caractérisé en ce que**, lorsque la deuxième zone perforée (11) comprend quatre ouvertures, deux lignes reliant la paire respective d'ouvertures opposées parmi les quatre ouvertures de la deuxième zone perforée (11) présentent une forme en croix ou toute autre forme diagonalement croisée dans l'espace tridimensionnel défini par une paroi de tube du cathéter principal de trachée (1).

7. Cathéter d'isolement de poumon à une seule lumière de type diviseur de gaz, configuré pour permettre une portée de structure de pont et une ventilation équilibrée commandée, selon la revendication 6, **caractérisé en ce que** la forme en croix présente une largeur et une hauteur égales l'une à l'autre ou la forme en croix est asymétrique en largeur et hauteur.

8. Cathéter d'isolement de poumon à une seule lumière de type diviseur de gaz, configuré pour permettre une portée de structure de pont et une ventilation équilibrée commandée, selon la revendication 1, **caractérisé en ce que** la paroi de chaque ouverture de la deuxième zone perforée (11) est disposée de façon inclinée par rapport à la paroi latérale du cathéter principal de trachée (1) et présente un angle d'inclinaison de l'un quelconque de 5°, 10°, 15°, 30°, 45° et 60° par rapport à un plan horizontal (1).

9. Cathéter d'isolement de poumon à une seule lumière de type diviseur de gaz, configuré pour permettre une portée de structure de pont et une ventilation équilibrée commandée, selon l'une des revendications 1 à 8, **caractérisé en ce qu'**un trou d'aspiration d'expectoration(12) est disposé à une extrémité inférieure du cathéter principal de trachée (1) et adjacent au cathéter bronchial (2), le trou d'aspiration d'expectoration (12) étant disposé de façon à être détourné de la première ouverture (21) et un diamètre du trou d'aspiration d'expectoration (12) étant identique à un diamètre de la première ouverture (21).

10. Cathéter d'isolement de poumon à une seule lumière de type diviseur de gaz, configuré pour permettre une portée de structure de pont et une ventilation équilibrée commandée, selon la revendication 9, **caractérisé en ce que** le trou d'aspiration d'expectoration (12) est configuré pour permettre le passage d'un tube spécifique d'aspiration d'expectoration (3) par celui-ci, le tube spécifique d'aspiration d'expectoration (3) étant pourvu de deux petits trous (31) et les deux petits trous (31) étant disposés opposés l'un à l'autre.

11. Cathéter d'isolement de poumon à une seule lumière de type diviseur de gaz, configuré pour permettre une portée de structure de pont et une ventilation équilibrée commandée, selon la revendication 10, **caractérisé en ce que** le tube spécifique d'aspiration d'expectoration (3) comprend une extrémité destinée à être pliée avec un angle de pliage de 90° à 150°, et, de préférence, un diamètre du tube spécifique d'aspiration d'expectoration (3) est un tiers du diamètre du cathéter bronchial (2).

12. Cathéter d'isolement de poumon à une seule lumière de type diviseur de gaz, configuré pour permettre une portée de structure de pont et une ventilation équilibrée commandée, selon l'une des revendications 1 à 11, **caractérisé en ce qu'**un premier ballon (4) est disposé sur une paroi extérieure du cathéter principal de trachée (1) et situé au-dessus de la deuxième zone perforée (11), le premier ballon (4) communiquant avec une première valve de gonflement (41) ;
un deuxième ballon (5) est disposé sur une paroi extérieure du cathéter bronchial (2), le deuxième ballon (5) communiquant avec une deuxième valve de gonflement (51) ;
un joint de manchette (6) est disposé à l'intérieur du cathéter bronchial (2) du cathéter d'isolement de poumon à une seule lumière de type diviseur de gaz, configuré pour permettre une portée de structure de pont et une ventilation équilibrée commandée, et le joint de manchette (6) communique avec une troisième valve de gonflement (61) via une commande de pression de ventilation (62) ; et
la commande de pression de ventilation (62) est configurée pour afficher une pression dans le joint de manchette (6) et régler une taille du joint de manchette (6) pour bloquer afin de commander un volume de flux de ventilation du cathéter bronchial (2) et de régler une distribution équilibrée de gaz dans les poumons.

13. Cathéter d'isolement de poumon à une seule lumière de type diviseur de gaz, configuré pour permettre une portée de structure de pont et une ventilation équilibrée commandée, selon la revendication 12, **caractérisé en ce que** la première ouverture (21) présente une surface S_{end}, et lesdites au moins deux deuxièmes ouvertures présentent une surface totale de S,
où S ≥ 1,5 Send.
